# EUROPEAN PATENT SPECIFICATION

(11) **EP 1 993 621 B1**
(45) Date of publication and mention of the grant of the patent: **17.08.2011**
(21) Application number: 07763255.2
(22) Date of filing: 06.02.2007
(51) Int. Cl.: A61L 15/22, A61L 17/12, A61L 27/26, A61L 29/04, A61L 31/04, C08L 67/04

(54) **TOUGHENED POLYLACTIC ACID POLYMERS AND COPOLYMERS**
VERSTÄRKTE POLYMILCHSÄUREPOLYMERE UND -COPOLYMERE
POLYMERES ET COPOLYMERES D'ACIDE POLYLACTIQUE DURCIS

(30) Priority: 07.02.2006 US 765808 P; 07.02.2006 US 765840 P; 12.05.2006 US 747144 P
(43) Date of publication of application: 26.11.2008
(73) Proprietor: Tepha, Inc., Lexington, Massachusetts 02421 (US)
(72) Inventor: RIZK, Said, Salem, NH 03079 (US); MARTIN, David, P., Arlington, MA 02474 (US); HO, Kicherl, Newton, MA 02464 (US); GANATRA, Amit, Attleboro, MA 02703 (US); WILLIAMS, Simon, F., Sherborn, MA 01770 (US)
(74) Representative: Wright, Andrew John
(86) International application number: PCT/US2007/003100
(87) International publication number: WO 2007/092417

(56) References cited:
- WO-A1-02/059201
- WO-A1-02/092691
- US-B1- 6 548 569

## Description

### Field of the Invention

The present invention generally relates to polymeric compositions that can be processed into various extruded as well as molded forms, including fibers, tubes, films, nonwovens, injection molded or thermoformed components, which products have substantially uniform physical properties, and physical and thermo-mechanical integrity. The compositions comprise polylactic acid polymers or copolymers and polymers or copolymers comprising 4-hydroxybutyrate.

### Background of the Invention

Polylactic acid (PLA) is an aliphatic polyester that can be prepared, for example, by direct condensation of lactic acid, azeotropic dehydrative condensation, and by ring-opening polymerization of lactide. In the latter case, the product is sometimes referred to as polylactide. The relative proportions of the optically active enantiomers, D- and L-lactic acids, which are incorporated into the polymer, determine the specific properties of PLA. Varying the enantiomer proportions can result in polymer compositions that are amorphous or up to about 40% crystalline, with glass transition temperatures (Tg) ranging from about 50°C to 80°C, and melting points (Tm) ranging from about 130°C to 180°C.

Elongation at break of poly-L-lactic acid (PLLA) is, however, typically just several percent. The polymer has a glass transition temperature well above room temperature, and therefore shaped objects of PLLA tend to be brittle and glassy at room temperature. Several methods have been used to increase the elongation at break of PLLA. Melt processing of the polymer, followed by orientation at temperatures above the glass transition temperature, can result in shaped objects with somewhat improved elongation to break. However, these objects are generally stiff due to the relatively high tensile modulus of the polymer.

Incorporation of D-lactic acid in combination with orientation can yield further improvement. For example, incorporation of D-lactic acid at a level of 2% in an oriented film increases the elongation at break to 5-10%. The latter may be further increased to 78-97% with incorporation of about 6% D-lactic acid. Orientation and incorporation of D-lactic acid has also been used to improve the toughness of fibers of PLA. Melt extruded fibers of PLA with elongation to break of 50-60% are commercially available under the trade name of Ingeo™ PLA (Cargill, MN). The modulus of these objects is however still relatively high.

Polymer scientists have also investigated blends of PLA with other polymers and additives to improve PLA properties, for example, to improve toughness and decrease the stiffness. For example, blends of PLA with other PLAs and copolymers, polycaprolactone, poly-3-hydroxybutyrate-co-3-hydroxyvalerate (PHBV), poly-R-3-hydroxybutyrate (PHB), poly-R;S-3-hydroxybutyrate, poly-3-hydroxyoctanoate, poly(hexamethylenesuccinate), poly(butylene succinate), poly(ethylene/butylene succinate), poly(ethylene oxide), poly(phosphazene), poly(sebacic anhydride), poly(vinyl alcohol), and poly(vinyl acetate) have all been reported (Tsuji, H., Polyesters, III, 4:129-177 (2002)). Blends of PLA comprising multiple components have also been reported. For example, U.S Patent No. 5,939,467 to Wnuk et al. discloses blends comprising PLA, polyhydroxyalkanoates, and polyurethane or polycaprolactone. These approaches have met with varying levels of success because many polymers are immiscible when blended, creating undesirable phase separation during processing, and/or such blends exhibit poor mechanical properties. These difficulties are exacerbated in the processing of fibers and films, where processing time is often much shorter.

It would therefore be desirable to identify polymers that could be blended with PLA and its copolymers, which provide a blend with improved toughness and lower stiffness.

It is therefore an object of this invention to provide toughened blends of PLA and its copolymers.

It is another object of this invention to provide blends of PLA and its copolymers with lower stiffness values.

It is still yet another object of this invention to provide blends of PLA and its copolymers with increased elongation to break.

It is yet another object of this invention to provide blends of PLA and its copolymers that can be processed into shaped objects, such as fibers, films, molded items, and nonwovens.

### Summary of the Invention

Toughened compositions of PLA and PLA copolymers are disclosed, which also have low tensile modulus values and greater elongation to break. These toughened compositions are prepared by blending PLA and PLA copolymers with poly-4-hydroxybutyrate, and copolymers thereof. Blending of poly-4-hydroxybutyrate with PLA and its copolymers has been found to impart advantageous properties to the resulting blend. These compositions, and objects formed from these compositions, have improved toughness and lower stiffness than polylactic acid polymers or copolymers alone.

### Brief Description of the Drawings

Figure 1 is the chemical structure of poly-4-hydroxybutyrate (P4HB, TephaFLEX^{®} biomaterial).
Figure 2 shows some of the known biosynthetic pathways for the production of P4HB. Pathway enzymes are: 1. Succinic semialdehyde dehydrogenase; 2. 4-hydroxybutyrate dehydrogenase; 3. diol oxidoreductase; 4. aldehyde dehydrogenase; 5. Coenzyme A transferase and 6. PHA synthetase.

### Detailed Description of the Invention

Toughened blends comprising PLA and its copolymers have been developed that have improved properties.

### I. Definitions

"Poly-4-hydroxybutyrate" as generally used herein means a homopolymer comprising 4-hydroxybutyrate units. It may be referred to herein as P4HB or TephaFLEX^{®} biomaterial (manufactured by Tepha, Inc., Cambridge, MA).

"Copolymers of poly-4-hydroxybutyrate" as generally used herein -means any polymer comprising 4-hydroxybutyrate with one or more different hydroxy acid units.

"Blend" as generally used herein means a physical combination of different polymers, as opposed to a copolymer comprised of two or more different monomers which are linked by polymerization.

"Toughness" means a property of a material by virtue of which it can absorb energy; the actual work per unit volume or unit mass of material that is required to rupture it. Toughness is usually proportional to the area under the load-elongation curve such as the tensile stress-strain curve. (Rosato's Plastics Encyclopedia and Dictionary, Oxford Univ. Press, 1993.)

"Stiffness" means a property of the material to resist a change in shape. For fibers, this is typically measured as the tensile modulus or Young's modulus and is the initial slope of the stress strain curve. For other shaped articles, like molded goods, the stiffness could relate to the ability of the material to resist bending.

"Elongation" or extensibility of a material means the amount of increase in length resulting from, as an example, the tension to break a specimen. It is expressed usually as a percentage of the original length. (Rosato's Plastics Encyclopedia and Dictionary, Oxford Univ. Press, 1993.)

"PLA" as used herein refers to a polymer comprising a lactic acid monomer unit. The polymer may be referred to as polylactic acid or polylactide. It may be a homopolymer or copolymer. The lactic acid repeating units can be L-lactic acid, D-lactic acid, or D,L-lactic acid. Copolymers may also comprise monomeric units other than lactic acid, such as, but not limited to, glycolic acid.

"Molecular weight" as used herein, unless otherwise specified, refers to the weight average molecular weight (Mw) as opposed to the number average molecular weight (Mn).

"Absorbable" as generally used herein means the material is broken down in the body and eventually eliminated from the body.

"Biocompatible" as generally used herein means the biological response to the material or device being appropriate for the device's intended application *in vivo.* Any metabolites of these materials should also be biocompatible.

"Compostable" as generally used herein means the ability of the material to undergo physical, chemical, thermal, and /or biological degradation in a municipal solid waste composting facility such that the material will break down into, or otherwise become part of, usable finished compost.

### II. Polymeric Compositions

### A. PLA Polymers

The polylactic acid or polylactide polymers (together referred to as "PLA") comprise a lactic acid monomeric unit, which may be L-lactic acid, D-lactic acid, or a mixture of D,L-lactic acid. Examples of commercially available PLA polymers include polylactic acid sold by Natureworks® (Cargill, MN), Lacea® sold by Mitsui Chemical (Tokyo, Japan), and Resomer sold by Boehringer Ingelheim (Ingelheim Am Rhein, Germany). In a preferred embodiment, the PLA is in a semi-crystalline form with at least 80 mole percent of the repeating unit being either L-lactide or D-lactide, and even more preferably 95 mole percent. If desired more than one PLA composition may be used. For example, two PLA polymers with different molecular weights or melting temperatures may be used. PLA copolymers may also comprise monomeric units other than lactic acid. A preferred PLA copolymer comprises glycolic acid, and is available commercially, for example, under the Vicryl^{®} trade name.

### B. Polymers Comprising 4-Hydroxybutyrate

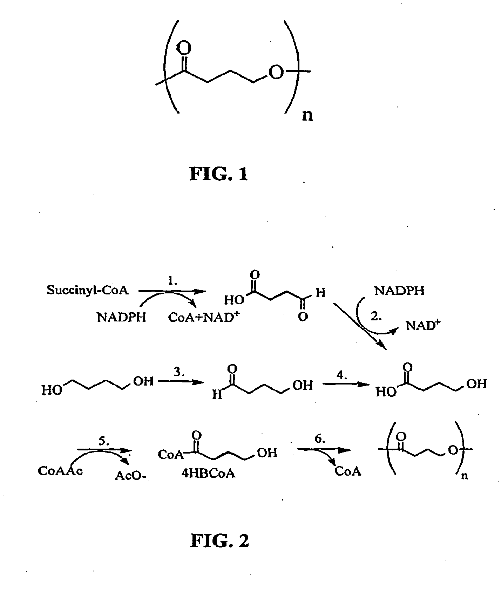

Tepha, Inc. of Cambridge, MA produces poly-4-hydroxybutyrate (P4HB) and copolymers thereof using transgenic fermentation methods. Poly-4-hydroxybutyrate is a strong, pliable thermoplastic polyester that is produced by a fermentation process, as described in U.S. Patent No. 6,548,569 to Williams et al.*.* Despite its biosynthetic route, the structure of the polyester is relatively simple (Figure 1). The polymer belongs to a larger class of materials called polyhydroxyalkanoates (PHAs) that are produced by numerous microorganisms (see: Steinbüchel A., et al. Diversity of Bacterial Polyhydroxyalkanoic Acids, FEMS Microbial. Lett. 128:219-228 (1995)). In nature these polyesters are produced as storage granules inside cells, and serve to regulate energy metabolism. They are also of commercial interest because of their thermoplastic properties, and relative ease of production. Several biosynthetic routes are currently known to produce P4HB, as shown in Figure 2. Chemical synthesis of P4HB has been attempted, but it has been impossible to produce the polymer with a sufficiently high molecular weight necessary for most applications (Hori, Y., et al., Polymer 36:4703-4705 (1995)).

Copolymers of P4HB include 4-hydroxybutyrate copolymerized with 3-hydroxybutyrate or glycolic acid (U.S. Patent Publication No. 20030211131 by Martin & Skraly, U.S. Patent No. 6,316,262 to Huisman et al.*,* and U.S. Patent No. 6,323,010 to Skraly et al.*).* Methods to control molecular weight of PHA polymers are disclosed by U.S. Patent No. 5,811,272 to Snell et al.

PHAs with degradation rates *in vivo* of less than one year are disclosed by U.S. Patent No. 6,548,569 to Williams et al. and WO 99/32536 by Martin et al. Applications of P4HB have been reviewed in Williams, S.F., et al., Polyesters, III, 4:91-127 (2002), and by Martin, D. et al. Medical Applications of Poly-4-hydroxybutyrate: A Strong Flexible Absorbable Biomaterial, Biochem. Eng. J. 16:97-105 (2003). Medical devices and applications of P4HB have also been disclosed by WO 00/56376 by Williams et al.

### C. Other Components

The toughened PLA compositions may comprise other materials in addition to the polymers described above, including plasticizers, nucleants, and compatibilizers. Non-limiting examples of plasticizers are disclosed by U.S Patent No. 6,905,987 to Noda et al. In addition, other components may be added to impart benefits such as, but not limited to, the following: stability including oxidative stability, brightness, color, flexibility, resiliency, workability, processibility (by addition of processing aids), viscosity modifiers, and odor control. Therapeutically, prophylactically or diagnostic agents may be added. Active components, such as drugs, and other biologically active substances may be incorporated, for example, for controlled release of the drugs or other substances. It may also be advantageous to incorporate contrast agents, radiopaque markers, or radioactive substances. For certain applications it may also be desirable to incorporate fillers, including materials such as titanium dioxide, calcium carbonate, hydroxyapatite, and tricalcium phosphate.

### II. Methods of Making Toughened Compositions of PLA and PLA Copolymers

In a preferred method, a toughened composition of a PLA polymer or PLA copolymer is prepared as follows. A PLA polymer or PLA copolymer is selected and compounded in a predetermined ratio with a P4HB polymer or copolymer using a twin screw extruder. If desired, other additives, such as plasticizers, nucleants, and compatibilizers may be added. Typically, the PLA polymer or copolymer will be present from 1% to 99%, by weight. The exact ratio of PLA to P4HB will be determined by the desired toughness and stiffness of the composition. Increasing the quantity of P4HB in the blend will generally decrease the tensile modulus (stiffness) and increase the elongation to break of the composition (and also any shaped object derived from the composition). The extrudate is cooled, and then cut into granules in a pelletizer. The granules obtained may then be subsequently processed by melt or solvent processing techniques.

PLA compositions toughened with P4HB polymers or copolymers are characterized by increased elongation to break, and decreased stiffness.

### III. Fabrication with Toughened PLA Compositions

The compositions possess properties that are desirable in both processing and the final product. For example, the properties would be desirable in processing by extrusion, injection/compression/blow molding, coating, spinning, blowing, thermoforming, laser cutting, thermal welding and calendaring processes. A person skilled in the art would recognize that the compositions are useful in any application where increased toughness, elongation and/or decreased stiffness is desired. The compositions may also provide other desired properties such as increased melt strength, impact and aging characteristics, and be useful in any application where these properties are desired.

The compositions may be used in commodity, industrial and medical applications. In the latter case, the compositions may be used to make partially or fully absorbable biocompatible medical devices. Such devices include sutures, suture fasteners, meniscus repair devices, rivets, tacks, staples, screws, bone plates and bone plating systems, biocompatible coatings, rotator cuff repair devices, surgical mesh, medical textiles and drapes, repair patches, slings, cardiovascular patches, tissue engineering scaffolds, vascular grafts, vascular closure devices, catheter balloons, anti-adhesion devices, drug delivery devices, embolization particles, orthopedic pins, adhesion barriers, stents (including coronary stents, peripheral stents, carotid stents, biliary stents, gastroenterology stents, urology stents, and neurology stents), embolization coils, guided tissue repair/regeneration devices, articular cartilage repair devices, nerve guides, tendon repair devices, intracardiac septal defect repair devices (including, but not limited to, atrial septal defect repair devices and PFO closure devices, and left atrial appendage (LAA) closure devices), pericardial patches, bulking and filling agents, vein valves, bone marrow scaffolds, meniscus regeneration devices, ligament and tendon grafts, ocular cell implants, spinal fusion cages, imaging devices, skin substitutes, dural substitutes, bone graft substitutes, bone dowels, wound dressings, and hemostats.

The devices can also be a shaped object used as or in packaging, personal hygiene products, bags, utensils, disposable and/or compostable articles, garments, surgical drapes, and gloves. The shaped object can be a disposable article, compostable article, packaging, personal hygiene product, textile, fiber, film, molded object, bag, utensil, garment, surgical drape, or glove.

The present invention will be further understood by reference to the following non-limiting examples.

### EXAMPLES

### Example 1: Preparation of a blend of PLA polymer and P4HB polymer

PLLA polymer (Resomer L 214 lot #1012474, Boehringer Ingelheim, Germany) with a viscosity of 5.9 dl/g (IV) was compounded with P4HB (poly-4-hydroxybutyrate, Mw 178,000, 2.3 dl/g (IV) (Tepha, Inc., Cambridge, MA) using a Leistritz 18 mm twin screw extruder (24:1 L/D). Compounding was undertaken at a temperature of 110°C at the feed, and 230°C at the die. The polymers were mixed in the molten state, extruded into large diameter filament, quenched into a water bath set at 5-20°C, and cut into 2-5 mm length pellets. The PLLA and P4HB were compounded in the following three different ratios by weight: PLLA(90%):P4HB(10%); PLLA(77.5%):P4HB (22.5%); and PLLA(10%):P4HB(90%).

### Example 2: Reparation of a blend of PLA polymer and P4HB polymer

PLLA polymer (Resomer L 214 lot #1002260, Boehringer Ingelheim, Germany) with a high intrinsic viscosity of 7.0 dl/g (IV) was dry mixed with P4HB (poly-4-hydroxybutyrate, Mw 170,000, 2.1 dl/g (IV) Tepha, Inc., Cambridge, MA) by tumble mixing at room temperature. The PLLA and P4HB were compounded in the following three different ratios by weight: PLLA(90%):P4HB(10%); PLLA(77.5%):P4HB (22.5%); and PLLA(10%):P4HB(90%).

### Example 3: Fiber Extrusion of PLA/P4HB blends

The blends prepared in Example 1 were extruded into monofilament fiber using the following method. The blends were dried under vacuum overnight to less than 0.01 % (w/w) water. Dried pellets of the blended polymers were fed into an extruder barrel of an AJA (Alex James Associates, Greer, SC) 3/4" single screw extruder (24:1 L:D, 3:1 compression) equipped with a Zenith type metering pump (0.16 cc/rev) and a die with a single hole spinneret (0.026", 2:1 L:D) under a blanket of nitrogen. The 4 heating zones of the extruder were set at 140°, 190°, 200° and 205°C. The block, metering pump and the die were maintained at a constant temperature, preferably 180-250°C. Pump discharge pressure was kept below 1500 psi by controlling the temperatures and the speed of the metering pump. The fiber spinning system was set up with a drop zone, air quench zone, a guide roll, three winders and a pickup. The fiber was oriented in-line with extrusion by drawing it in a multi-stage process to provide improved physical properties with stretch ratios of 1 to 11X. The resulting spun extrudate filament was free from all melt irregularities. To provide optimal physical properties to fiber, the extrudate filament was then drawn through multistage orientation in a heated tube or heated oven or hot water, which was maintained at a temperature above the softening temperature of the extrudate filament and then quenched in a cold water bath without the filament touching any surface until it is sufficiently cooled.

Oriented monofilament fiber produced according to the procedure of Example 3 was tested on a MTS Mechanical Analyzer. The results are shown in Table 1 below.

**Table 1: Properties of PLLA:P4HB Blends at different ratios versus PLLA**

| Sample (% by weight) | Diameter (mm) | Break Load (kgf) | Break Stress (kgf/mm²) | Elongation at Break (%) | Young's Modulus (kgf/mm²) | Knot Load (kgf) |
|---|---|---|---|---|---|---|
| P4HB(90%):PLLA (10%) | 0.309 | 5.05 | 67.4 | 24.3 | 170.9 | 3.01 |
| P4HB(22.5%):PLLA (77.5%) | 0.312 | 2.92 | 36.9 | 17.0 | 270.4 | 2.07 |
| P4HB(10%):PLLA (90%) | 0.382 | 4.02 | 35.2 | 16.7 | 302.1 | 2.89 |
| Reference: PLLA(100%) | 0.106 | 0.6 | 66.0 | 3.0 | 700.0 | Nd |

It is evident from Table 1 that blending P4HB with PLLA increases the toughness of the PLLA (elongation at break increases), and decreases Young's modulus. The extent of these changes increases with increasing weight percentage of P4HB in the blend.

Note: The fibers in Table 1 have been oriented to increase the tensile strength of the fiber. Much of the elongation to break is reduced during the orientation process. (Higher elongation to break (>200%) for partially oriented tubes is disclosed in Example 4.)

### Example 4: Tube Extrusion of PLA/P4HB blends

The blends prepared in Example 1 and Example 2 were dried as described in Example 3 and extruded into tubes with internal diameters (ID) of 1-1.4 mm, and outer diameters (OD) of 1.3-1.7 mm using the following equipment, 1.25" 3 heat zone, 24:1 L/D, 3:1 compression horizontal extruder connected to conventional tubing die with an air inlet port to control the ID of tubes. A water trough for tube quenching, dual-plane laser gauge to measure OD in two planes and a puller were also used. Extruder temperatures were controlled at 120°C for the first zone, 240°C for the second zone, 265°C for the third zone and 248°C for the die. Screw speed was controlled between 10 - 40 rpm and the die pressure was maintained at 1500 psi. During the extrusion the quench trough was maintained at 10°C and process air at 10-30 inch H₂O pressure was used to obtain the intended tubing ID.

The extruded tubes were tested on a MTS Mechanical Analyzer, the Mw's of the tubes were analyzed by Gel Permeation Chromatography (GPC) and the results are shown in Table 2.

**Table 2. Properties of tubing melt extruded from P4HB:PLLA blends (22.5:77.5% by weight).**

| Mixing Method | Mw by GPC g/mol | OD (mm) | ID (mm) | Elongation To Break (%) | Tensile Strength (MPa) |
|---|---|---|---|---|---|
| Twin Screw | 285,000 | 1.547±0.042 | 1.097 | 281±69 | 33.5 |
| Dry Blend | 344,000 | 1.613±0.029 | 1.363 | 206±92 | 47.0 |

## Claims

1. A polymer composition comprising a polymer blend comprising polylactic acid polymer or copolymer and a polymer or copolymer comprising 4-hydroxybutyrate, wherein the elongation to break of the composition is greater than the elongation to break of a composition comprising the polylactic acid polymer or copolymer alone.

2. The composition of claim 1 wherein the modulus of the composition is less than the modulus of a composition comprising the polylactic acid polymer or copolymer alone.

3. The composition of claim 1 comprising a poly-4-hydroxybutyrate homopolymer.

4. The composition of claim 1 comprising poly-3-hydroxybutyrate-co-4-hydroxybutyrate.

5. The composition of claim 1 comprising a polyhydroxy acid selected from the group consisting of poly-L-lactic acid, poly-D-lactic acid, and poly-D,L-lactic acid.

6. The composition of claim 5 wherein the composition comprises polylactic-co-glycolic acid.

7. The composition of claim 1 further comprising one or more additives selected from the group consisting of plasticizers, nucleants, compatibilizers, therapeutic, prophylactic or diagnostic agents, radiolabelled substances, imaging agents, radiopaque markers, contrast agents, anti-oxidants, dyes, viscosity modifiers, and odor control agents.

8. A method of producing a shaped object comprising melting a polymer composition as defined by claim 1, and producing a shaped object therefrom by extrusion, molding, coating, spinning, blowing, thermoforming or calendaring processes, or combinations of these processes.

9. The method of claim 8 wherein the shaped object is a medical device selected from the group consisting of sutures, suture fasteners, meniscus repair devices, rivets, tacks, staples, screws, bone plates and bone plating systems, biocompatible coatings, rotator cuff repair devices, surgical mesh, medical textiles or drapes, repair patches, slings, cardiovascular patches, tissue engineering scaffolds, vascular grafts, vascular closure devices, catheter balloons, and-adhesion devices, drug delivery devices, embolization particles, orthopedic pins, adhesion barriers, stents, embolization coils, guided tissue repair/regeneration devices, articular cartilage repair devices, nerve guides, tendon repair devices, intracardiac septal defect repair devices, pericardial patches, bulking and filling agents, vein valves, bone marrow scaffolds, meniscus regeneration devices, ligament and tendon grafts, ocular cell implants, spinal fusion cages, imaging devices, skin substitutes, dural substitutes, bone graft substitutes, bo ne dowels, wound dressings, and hemostats.

10. The method of claim 8 wherein the shaped object can be used as or in packaging, personal hygiene products, bags, utensils, disposable or compostable articles, garments, surgical drapes, and gloves.

11. A medical device comprising a polymer composition as defined by Claim 1.

12. The medical device of claim 11 wherein the device is a suture, suture fastener, meniscus repair device, rivet, tack, staple, screw, bone plate, bone plating system, biocompatible coating, rotator cuff repair device, surgical mesh, medical textile, repair patch, sling, cardiovascular patche, tissue engineering scaffold, vascular graft, vascular closure device, catheter balloon, anti-adhesion device, drug delivery device, embolization particle, orthopedic pin, adhesion barrier, stent (including coronary stents, peripheral stents, carotid stents, biliary stents, gastroenterology stents, urology stents, and neurology stents), embolization coil, guided tissue repair/regeneration device, articular cartilage repair device, nerve guide, tendon repair device, intracardiac septal' defect repair device (including, but not limited to, atrial septal defect repair devices and PFO closure devices, and left atrial appendage (LAA) closure devices), pericardial patch, bulking and filling agent, vein valves, bone marrow scaffold, meniscus regeneration device, ligament graft, tendon graft, ocular cell implant, spinal fusion cage, imaging device, skin substitute, dural substitute, bone graft substitute, bone dowel, wound dressing, or a hemostat.

13. A shaped object comprising a polymer composition as defined by Claim 1.

14. The shaped object of claim 13 where the object is a disposable article, compostable article, packaging, personal hygiene product, textile, fiber, film, molded object, bag, utensil, garment, surgical drape, or glove.

## Patentansprüche

1. Polymerzusammensetzung, die ein Polymerblend umfasst, das ein Polymilchsäurepolymer oder -copolymer und ein 4-Hydroxybutyrat umfassendes Polymer oder Copolymer umfasst, wobei die Bruchdehnung der Zusammensetzung größer als die Bruchdehnung einer Zusammensetzung ist, die das Polymilchsäurepolymer oder -copolymer allein umfasst.

2. Zusammensetzung nach Anspruch 1, wobei der Modul der Zusammensetzung kleiner als der Modul einer Zusammensetzung ist, die das Polymilchsäurepolymer oder -copolymer allein umfasst.

3. Zusammensetzung nach Anspruch 1, die ein Poly-4-hydroxybutyrat-Homopolymer umfasst.

4. Zusammensetzung nach Anspruch 1, die Poly-3-hydroxybutyrat-co-4-hydroxybutyrat umfasst.

5. Zusammensetzung nach Anspruch 1, die eine Polyhydroxysäure umfasst, die aus der aus Poly-L-milchsäure, Poly-D-milchsäure und Poly-D,L-milchsäure bestehenden Gruppe ausgewählt ist.

6. Zusammensetzung nach Anspruch 5, wobei die Zusammensetzung Polymilch-coglycolsäure umfasst.

7. Zusammensetzung nach Anspruch 1, die ferner ein oder mehrere Additiv(e) umfasst, das bzw. die aus der Gruppe ausgewählt ist bzw. sind, die besteht aus Weichmachern, Nukleierungsmitteln, Kompatibilisatoren, therapeutischen, prophylaktischen oder diagnostischen Mitteln, radioaktiv markierten Substanzen, Agenzien für eine bildgebende Darstellung, radiopaken Markern, Kontrastmitteln, Antioxidanzien, Farbstoffen, Viskositätsmodifikatoren und Geruchsbekämpfungsmitteln.

8. Verfahren zur Herstellung eines geformten Gegenstands, das das Schmelzen einer Polymerzusammensetzung, wie sie im Anspruch 1 definiert ist, und das Herstellen eines geformten Gegenstands aus dieser mittels Extrusions-, Form-, Beschichtungs-, Spinn-, Blas-, Thermoform- oder Kalanderprozessen oder Kombinationen dieser Prozesse umfasst.

9. Verfahren nach Anspruch 8, wobei der geformte Gegenstand ein Medizinprodukt ist, das aus der Gruppe ausgewählt ist, die besteht aus Nähten, Nahtbefestigern, Vorrichtungen für die Meniskusreparatur, Nieten, Stiften, Klammern, Schrauben, Knochenplatten und Systemen für die Knochenbeplattung, biokompatiblen Beschichtungen, Vorrichtungen für die Reparatur der Rotatorenmanschette, chirurgischen Netzen, medizinischen Textilien oder Abdecktüchern, Reparaturpatches, Schlingen, kardiovaskulären Patches, Gerüsten für ein Gewebeengineering, Gefäßersatzprodukten, Vorrichtungen für einen Gefäßverschluss, Katheterballonen, Adhäsionsschutzprodukten, Vorrichtungen für eine Arzneimittelzufuhr, Embolisationspartikeln, orthopädischen Stiften, Adhäsionsbarrieren, Stents, Embolisationscoils, Vorrichtungen für eine gelenkte Gewebereparatur/regeneration, Vorrichtungen für die Gelenkknorpelreparatur, Leitschienen für Nerven, Vorrichtungen für die Sehnenreparatur, Vorrichtungen für die Reparatur intrakardialer Septumdefekte, Perikardpatches, Füllmitteln und Füllstoffen, Venenklappen, Knochenmarksgerüsten, Vorrichtungen für die Meniskusregeneration, Ligament- und Sehnenersatzprodukten, Augenzellenimplantaten, Spondylodesekäfigen, Vorrichtungen für eine bildgebende Darstellung, Hautersatzprodukten, Duraersatzprodukten, Knochenersatzprodukten, Knochendübeln, Wundverbänden und Hämostaten.

10. Verfahren nach Anspruch 8, wobei der geformte Gegenstand als Verpackung, Körperpflegeprodukt, Beutel, Utensil, Wegwerfartikel oder kompostierbarer Artikel, Bekleidungsartikel, Operationsabdecktuch und Handschuh oder in diesen verwendet werden kann.

11. Medizinprodukt, das eine Polymerzusammensetzung, wie sie durch Anspruch 1 definiert ist, umfasst.

12. Medizinprodukt nach Anspruch 11, wobei die Vorrichtung ist ein(e) Naht, Nahtbefestiger, Vorrichtung für die Meniskusreparatur, Niete, Stift, Klammer, Schraube, Knochenplatte, System für die Knochenbeplattung, biokompatible Beschichtung, Vorrichtung für die Reparatur der Rotatorenmanschette, chirurgisches Netz, medizinisches Textil, Reparaturpatch, Schlinge, kardiovaskulärer Patch, Gerüst für ein Gewebeengineering, Gefäßersatzprodukt, Vorrichtung für einen Gefäßverschluss, Katheterballon, Adhäsionsschutzprodukt, Vorrichtung für eine Arzneimittelzufuhr, Embolisationspartikel, orthopädischer Stift, Adhäsionsbarriere, Stent, (einschließlich Koronarstents, peripheren Stents, Karotisstents, Gallenstents, gastroenterologischen Stents, urologischen Stents und neurologischen Stents), Embolisationscoil, Vorrichtung für eine gelenkte Gewebereparatur/regeneration, Vorrichtung für die Gelenkknorpelreparatur, Leitschiene für Nerven, Vorrichtung für die Sehnenreparatur, Vorrichtung für die Reparatur intrakardialer Septumdefekte (einschließlich, ohne jedoch auf diese beschränkt zu sein, Vorrichtungen für die Reparatur von Atriumseptumdefekten und Vorrichtungen für den PFO-Verschluss und Vorrichtungen für den Verschluss des linken Vorhofohrs (LAA-Verschluss)), Perikardpatch, Füllmittel und Füllstoff, Venenklappe, Knochenmarksgerüst, Vorrichtung für die Meniskusregeneration, Ligamentersatzprodukt, Sehnenersatzprodukt, Augenzellenimplantat, Spondylodesekäfig, Vorrichtung für eine bildgebende Darstellung, Hautersatzprodukt, Duraersatzprodukt, Knochenersatzprodukt, Knochendübel, Wundverband oder ein Hämostat.

13. Geformter Gegenstand, der eine Polymerzusammensetzung, wie sie durch Anspruch 1 definiert ist, umfasst.

14. Geformter Gegenstand nach Anspruch 13, wobei der Gegenstand ein Wegwerfartikel, ein kompostierbarer Artikel, eine Verpackung, ein Körperpflegeprodukt, ein Textil, eine Faser, eine Folie, ein gemoldeter Gegenstand, ein Beutel, ein Utensil, ein Bekleidungsartikel, ein Operationsabdecktuch oder ein Handschuh ist.

## Revendications

1. Composition de polymères comprenant un mélange de polymères comprenant un polymère ou copolymère de poly(acide lactique) et un polymère ou copolymère comprenant du 4-hydroxybutyrate, où l'allongement à la rupture de la composition est supérieur à l'allongement à la rupture d'une composition comprenant le polymère ou copolymère de poly(acide lactique) seul.

2. Composition selon la revendication 1, dans laquelle le module de la composition est inférieur au module d'une composition comprenant le polymère ou copolymère de poly(acide lactique) seul.

3. Composition selon la revendication 1, comprenant un homopolymère de poly-4-hydroxybutyrate.

4. Composition selon la revendication 1, comprenant du poly-3-hydroxybutyrate-co-4-hydroxybutyrate.

5. Composition selon la revendication 1, comprenant un polyhydroxy acide choisi dans le groupe constitué par le poly(acide L-lactique), le poly(acide D-lactique), et le poly(acide D,L-lactique).

6. Composition selon la revendication 5, dans laquelle la composition comprend du poly(acide lactique-co-glycolique).

7. Composition selon la revendication 1, comprenant en outre un ou plusieurs additifs choisis dans le groupe constitué par les plastifiants, les agents de nucléation, les agents de compatibilisation, les agents thérapeutiques, prophylactiques ou diagnostiques, les substances marquées, les agents d'imagerie, les marqueurs radioopaques, les agents de contraste, les antioxydants, les colorants, les modificateurs de viscosité et les agents de lutte contre les odeurs.

8. Procédé de production d'un objet mis en forme comprenant la fusion d'une composition de polymères telle que définie dans la revendication 1, et la production d'un objet mis en forme à partir de celle-ci par des procédés d'extrusion, de moulage, de revêtement, de filature, de soufflage, de thermoformage ou de calandrage, ou des combinaisons de ces procédés.

9. Procédé selon la revendication 8, dans lequel l'objet mis en forme est un dispositif médical choisi dans le groupe constitué par les sutures, les fixations pour suture, les dispositifs de réparation du ménisque, les rivets, les colles, les agrafes, les vis, les lames osseuses et les systèmes de lame osseuse, les revêtements biocompatibles, les dispositifs de réparation de coiffe des rotateurs, les mèches chirurgicales, les textiles ou champs médicaux, les pièces de réparation, les écharpes, les pièces cardiovasculaires, les structures d'ingénierie tissulaire, les greffes vasculaires, les dispositifs d'obturation vasculaire, les ballonnets de cathéter, les dispositifs anti-adhérence, les dispositifs d'administration de médicament, les particules d'embolisation, les broches orthopédiques, les barrières d'adhérence, les endoprothèses vasculaires, les spirales d'embolisation, les dispositifs guidés de réparation/génération tissulaire, les dispositifs de réparation des cartilages articulaires, les guides nerveux, les dispositifs de réparation des tendons, les dispositifs de réparation des défauts du septum intracardiaque, les pièces péricardiques, les agents de gonflage et de remplissage, les valvules veineuses, les structures de soutien de la moelle osseuse, les dispositifs de régénération du ménisque, les greffes de ligament et de tendon, les implants de cellules oculaires, les cages de fusion de vertèbres, les dispositifs d'imagerie, les substituts de peau, les substituts de dure-mère, les substituts de greffe osseuse, les ancrages osseux, les pansements pour plaies, et les dispositifs hémostatiques.

10. Procédé selon la revendication 8, dans lequel l'objet mis en forme peut être utilisé en tant qu'emballage, produit d'hygiène intime, sachet, ustensile, article jetable ou compostable, vêtement, champ chirurgical et gant, ou intégré dans ceux-ci.

11. Dispositif médical comprenant une composition de polymères telle que définie par la revendication 1.

12. Dispositif médical selon la revendication 11, dans lequel le dispositif est une suture, une fixation pour suture, un dispositif de réparation du ménisque, un rivet, une colle, une agrafe, une vis, une lame osseuse et un système de lame osseuse, un revêtement biocompatible, un dispositif de réparation de coiffe des rotateurs, une mèche chirurgicale, un textile médical, une pièce de réparation, une écharpe, une pièce cardiovasculaire, une structure d'ingénierie tissulaire, une greffe vasculaire, un dispositif d'obturation vasculaire, un ballonnet de cathéter, un dispositif anti-adhérence, un dispositif d'administration de médicament, une particule d'embolisation, une broche orthopédique, une barrière d'adhérence, une endoprothèse vasculaire (incluant les endoprothèses coronariennes, les endoprothèses périphériques, les endoprothèse carotidiennes, les endoprothèses biliaires, les endoprothèses gastroentérologiques, les endoprothèses urologiques et les endoprothèses neurologiques), une spirale d'embolisation, un dispositif guidé de réparation/génération tissulaire, un dispositif de réparation des cartilages articulaires, un guide nerveux, un dispositif de réparation des tendons, un dispositif de réparation des défauts du septum intracardiaque (comprenant, sans limitation, les dispositifs de réparation d'un défaut du septum interauriculaire et les dispositifs d'obturation PFO, et les dispositifs d'obturation de l'appendice auriculaire gauche (LAA)), une pièce péricardique, un agent de gonflage et de remplissage, une valvule veineuse, une structure de soutien de la moelle osseuse, un dispositif de régénération du ménisque, une greffe de ligament, une greffe de tendon, un implant de cellules oculaires, une cage de fusion de vertèbres, un dispositif d'imagerie, un substitut de peau, un substitut de dure-mère, un substitut de greffe osseuse, un ancrage osseux, un pansement pour plaies, ou un dispositif hémostatique.

13. Objet mis en forme comprenant une composition de polymères telle que définie par la revendication 1.

14. Objet mis en forme selon la revendication 13, dans lequel l'objet est un article jetable, un article compostable, un emballage, un produit d'hygiène intime, un textile, une fibre, un film, un objet moulé, un sachet, un ustensile, un vêtement, un champ chirurgical ou un gant.
